# EUROPEAN PATENT SPECIFICATION

(11) **EP 0 693 134 B1**
(45) Date of publication and mention of the grant of the patent: **25.06.1997**
(21) Application number: 94909110.2
(22) Date of filing: 03.03.1994
(51) Int. Cl.: C12P 41/00, C12P 7/40

(54) **ARYLALKANOIC ACID RESOLUTION**
ARYLALKANSÄURETRENNUNG
RESOLUTION DE L'ACIDE ARYLALCANOIQUE

(30) Priority: 03.03.1993 GB 9304256
(43) Date of publication of application: 24.01.1996
(73) Proprietor: LABORATORIOS MENARINI S.A., 08912 Badalona (ES)
(72) Inventor: WARNECK, Julie Belinda Hazel, Sawtree, Huntingdon PE17 5UU (GB); WISDOM, Richard Anthony, Cambs CB4 4NP (GB)
(74) Representative: Minoja, Fabrizio
(86) International application number: EP9400630
(87) International publication number: WO9420633

(56) References cited:
- EP-A- 0 233 656
- EP-A- 0 407 033
- WO-A-93/04189
- WO-A-93/25703
- WO-A-93/25704

## Description

### Field of the Invention

This invention relates to arylalkanoic acid resolution, particularly to 2-(3-benzoylphenyl)propionic acid (Ketoprofen) resolution.

### Background of the Invention

A number of 2-arylpropionic acids are well known as anti-inflammatory agents. Examples include ibuprofen, naproxen and ketoprofen. These compounds are chiral, and it is now well established that their major therapeutic activity resides in the (S)-enantiomer.

Several methods for obtaining the (S)-enantiomer, free of contaminating (R)-enantiomer, are known. These include asymmetric chemical syntheses and chemical resolution such as the stoichiometric crystallisation of diastereomeric salts formed with various chiral amines.

An alternative approach is biocatalytic, using a biocatalyst to selectively hydrolyse an ester of the 2-arylpropionic acid. Providing a biocatalyst with the correct stereospecificity can be identified, a reaction mixture containing the unreacted ester of one enantiomer plus the acid product of the other enantiomer can be obtained. Separation and recovery of the product are then relatively facile. Unreacted ester can be racemised and reused in a further reaction, thereby ensuring almost complete conversion of the racemic substrate to the required single enantiomer product.

EP-A-0227078 describes the use of several extracellular, commercially-available microbial lipases in such biocatalytic resolutions. In general, however, large amounts of enzyme were required and the reaction took 2-6 days. Such reactions would therefore be expensive to operate. The best enzyme powder identified was that from Candida cylindracea (also known as Candida rugosa); however, in EP-A-0407033, it was shown that this preparation, as well as having low activity, contained more than one enzyme with esterase activity. Further, in order to obtain ketoprofen with a high ee, it was necessary to purify the preparation.

EP-A-0233656 describes the isolation and cloning of an esterase gene from Bacillus thai. This enzyme is shown to selectively hydrolyse the ethyl and methyl esters of both naproxen and ibuprofen to give the (s)-acid of respective compounds. It is also shown that cloning of the enzyme resulted in a preparation giving a higher ee product, as a result of minimising other enzyme side-activities.

WO-A-9323547 describes a number of strains which also produce an esterase which selectively hydrolyses naproxen esters. The best strain found was Zopfiella latipes, the esterase from which had been cloned.

WO-A-9304189 describes an organism, Trichosporon sp, that is able to selectively hydrolyse the (S)-enantiomer of ethyl ketoprofen to give an (S)-acid product having an ee of >90%. The enzyme that carries out this biotransformation is intracellular. As stable cell-free preparations have proved difficult to obtain, it is difficult to increase biocatalytic activities by gene cloning or classical mutagenesis.

An object behind the present invention has been to obtain a biocatalyst which has good activity against different ketoprofen esters, which gives good ee ketoprofen acid product, and which would be suitable for cloning and hyper-expression, e.g. in E. coli, to enable biocatalyst costs to be kept to a minimum.

### Summary of the Invention

Surprisingly, a screen of microorganisms from a range of different sources showed that the ascomycete Ophiostoma novo-ulmi (also known as Ceratocystis ulmi) produced an intracellular hydrolytic enzyme which could carry out the desired reaction. Further tests demonstrated that, unlike the strain disclosed in WO-A-9304189, stable cell-free activity could be obtained and that therefore it was suitable for cloning and potential use in cell-free biotransformations. Ophiostoma novo-ulmi is a plant pathogen known to be the virulent causative agent of Dutch Elm disease. It is known to be closely related to less virulent strains of Ophiostoma ulmi and also to O. piceae.

Following the primary discovery underlying the present invention, it has been found that stereospecific esterase activity is present in alternative strains of Ophiostoma novo-ulmi in Ophiostoma ulmi (also sometimes known as Ceratocystis ulmi) and Ophiostoma piceae (also sometimes known as Ceratocystis picea) and from isolates collected from various locations in the USA, Europe, the Middle East and Uzbekistan. In addition, alternative strains of Ceratocystis sp. obtained from the IMI, Egham, Surrey, UK have been obtained and screened for activity. It has been discovered that the activity is also present in strains C. coronata (e.g. IMI 176533), C. ips (e.g. IMI 212114), C. tetropii (e.g. IMI 212117), C. cainii (e.g. IMI 176523), C. arborea (e.g. IMI 176529) and C. stenoceras (e.g. IMI 268494). The activity appears therefore to be widespread amongst a range of alternative Ceratocystis strains collected from different locations in the world.

A strain of the original isolate screened, herein described as AJ3, was deposited at the IMI on 15th February 1993, with the accession number IMI 356050, under the terms of the Budapest Treaty. This strain provides a good example of the activity; however, given the widespread nature of the activity in a wide variety of related strains, the scope of the invention is not intended to be limited to this particular organism. The invention also extends to the use of enzymatic activity isolated from such organisms, by any suitable technique.

### Description of the Invention

In accordance with the present invention, microorganisms of the genus Ophiostoma and their enzymatic activity can be used to hydrolyse the racemic alkyl ester of ketoprofen stereo-selectively, to yield the acid substantially enriched in the (S)-enantiomer e.g. to 93-96% enantiomeric excess, or more, at 15-25% conversion and leave residual ester enriched with the (R)-enantiomer. The reaction can be conducted on any mixture of enantiomers of the ester, although that mixture will usually be the racemate. The alkyl group of the ester is preferably of 1 to 3 carbon atoms. The reaction is preferably conducted at pH 8-11, more preferably 9.5-10.5, and most preferably about 10. An organic solvent such as cyclohexane is usually employed.

Standard chemical procedures may then be used to separate the acid product from the ester. Where necessary, the optical purity of the acid can be improved by the use of a chiral amine such as (R)-α-methylbenzylamine. The ester that is left, unconverted, can be racemised, for further use.

The following Examples illustrate the invention.

### Example 1 Biotransformation of racemic ethyl Ketoprofen by AJ3

The following medium was used for the growth of the organism:

| | |
|---|---|
| (NH₄)₂SO₄ (g/l) | 0.5 |
| MgSO₄.7H₂O (g/l) | 0.25 |
| CaCl₂.2H₂O (g/l) | 0.1 |
| KH₂PO₄ (g/l) | 8.0 |
| Yeast Extract (g/l) | 10.0 |
| Glucose (g/l) | 5.0 |
| Trace element solution (µl/l) | 100 |
| pH (with NaOH) | 6.5 |

The trace element solution used was:

| | |
|---|---|
| CaCl₂.2H₂O (g/l) | 3.57 |
| ZnO (g/l) | 2.0 |
| CuCl₂.2H₂O (g/l) | 0.85 |
| Na₂MoO₄.2H₂O (g/l) | 4.8 |
| MnCl₂.4H₂O (g/l) | 2.0 |
| FeCl₃.6H₂O (g/l) | 5.4 |
| H₃BO₄ (g/l) | 0.3 |
| CoCl₂.6H₂O (g/l) | 2.4 |
| HCl (ml/l) | 250 |

Cells of AJ3 were inoculated from a malt extract agar plate into 30 ml growth medium in a 250 ml baffled flask. This was shaken for 30 hours at 23°C and then 2.5 ml transferred into a second 250 ml baffled flask containing 30 ml of medium. Following growth for 40 hours, at 23°C with shaking, racemic ethyl ketoprofen was added to the broth to a concentration of 20 g/l. The biotransformation was then allowed to proceed for 120 hours, after which HPLC analysis showed there to have been 18.2% hydrolysis of the added ester. The enantiomeric excess of the ketoprofen acid formed was found to be 96% in favour of the (S)-enantiomer.

### Example 2 Effect of organic solvent

A crude enzyme solution was prepared, using the same growth medium as in Example 1, minus glucose and at a pH of 6.0. Cells of AJ3 were inoculated into 100 ml medium in a 1 litre shake flask. This culture was grown with shaking at 30°C for 48 hours. 10 ml was then transferred into 90 ml fresh medium in a 1 litre shake flask and grown for a further 8 hours at 30°C with shaking. This flask was used to inoculate a 2.8 1 laboratory fermenter with 1.5 1 medium plus 5 g/l glucose and 0.5 ml/l of a silicone/PPG-based antifoam (XFO371, Ivanhoe Chemicals, Ill., USA). This fermenter was operated for 48 hours with agitation and aeration, controlled to maintain a DOT >50% air saturation and temperature and pH at 30°C and 6.0 respectively.

At the completion of the fermentation the cells were harvested by centrifugation and the pellet resuspended at 10% w/v (based on wet cell weight) in lysis buffer, i.e. 0.1M sodium carbonate in 5% Triton X-100 aqueous solution. Lysis was carried out overnight at 8°C with shaking after which the enzyme activity-containing lysate was separated from the cell debris by centrifugation.

Racemic ethyl ketoprofen was dissolved in each of four organic solvents: toluene, cyclohexane, methanol and MTBE (methyl t-butyl ether) to a concentration of 20 g/l. 2 ml of each of these solutions was added to 5 ml aliquots of crude enzyme solution in glass vials. A control aqueous biotransformation containing 5 ml crude enzyme solution, 400 µl 50% racemic ethyl ketoprofen, 0.5% w/v Tween 80 stock solution and 2.5% w/v Triton x-100 was also prepared in a glass vial. All samples were reacted with shaking for 48 hours at 25°C. Analysis of the aqueous phase of each reaction mixture gave the results shown in Table 1.

**Table 1**

| HYDROLYSIS OF RACEMIC ETHYL KETOPROFEN IN 2-PHASE BIOTRANSFORMATIONS | | |
|---|---|---|
| SAMPLE SOLVENT | ACID PRODUCED (mg.ml⁻¹) | CONVERSION (%) |
| AQUEOUS | 2.3 | 6.2 |
| TOLUENE | 0.1 | 1.3 |
| CYCLOHEXANE | 1.1 | 14.1 |
| METHANOL | 0.4 | 4.6 |
| MTBE | 0.7 | 8.3 |

It is observed that the biotransformation works to a certain extent in the presence of cyclohexane or MTBE as solvents, but is quite strongly inhibited by toluene under these conditions.

### Example 3 Effect of the alkyl group

Various esters of racemic ketoprofen were dissolved in cyclohexane to a concentration of 20 g/l. The esters used were methyl, ethyl, n-propyl, n-butyl, n-pentyl, n-hexyl. In the case of methyl ester, a suspension was prepared due to low solubility of ester in cyclohexane.

To 5 ml aliquots of crude enzyme solution in glass vials was added 2 ml of each of the six racemic ketoprofen ester solutions above. An aqueous control biotransformation was also prepared in a glass vial containing 5 ml crude enzyme solution. 200 µl 50% racemic ethyl ketoprofen, 0.5% Tween 80 stock solution and 2.5% w/v Triton x-100. Reactions were conducted with shaking for 24 hours at 25°C. HPLC analysis of the aqueous phase of each biotransformation gave the results shown in Table 2.

**Table 2**

| HYDROLYSIS OF RACEMIC KETOPROFEN ESTERS IN 2 PHASE BIOTRANSFORMATIONS. | | | |
|---|---|---|---|
| KETOPROFEN ESTER | ACID PRODUCED (mg.ml⁻¹⁾ | CONVERSION (%) | EE (%)(S)-enantiomer |
| AQUEOUS CONTROL | 4.6 | 22.9 | 90 |
| METHYL | 1.7 | 42.3 | 90 |
| ETHYL | 1.1 | 27.5 | 100 |
| n-PROPYL | 1.0 | 25.6 | 86 |
| n-BUTYL | 0.3 | 8.0 | N/D |
| n-PENTYL | 0.18 | 4.4 | N/D |
| n-HEXYL | 0.09 | 2.3 | N/D |
| N/D = not determined owing to the low acid accumulated. | | | |

### Example 4 Preparation of AJ3 lysate

The AJ3 isolate was grown to the 500 litre scale using the following methodology.

AJ3 was inoculated into 100 ml medium (as Example 1) in a 1 litre point baffled flask. Following growth for 48 hours at 25°C with orbital shaking (300 rpm, 25 mm throw), 1 ml was transferred to each of three 1 litre baffled flasks each containing 100 ml medium. The cultures were then grown for a further 48 hours under similar conditions before being combined and used to inoculate a 750 l fermenter containing 500 l medium.

The following medium was used:

| | |
|---|---|
| MgSO₄.7H₂O | 0.4 g/l |
| CaCl₂.2H₂O | 0.1 g/l |
| KH₂PO₄ | 8.0 g/l |
| Yeast Extract | 30.0 g/l |
| Trace Element Solution | 200 µl/l (as Example 1) |
| Antifoam (XFO-371) | 0.5 ml/l |
| pH (with NaOH) | 6.0 |

This medium was sterilised at 121°C for 50 minutes prior to the addition of 15 1 of 50% w/v sterile glucose solution.

Control conditions were: temperature 25°C, pH at 6.0 phosphoric acid or sodium hydroxide addition and DOT ≥50% of air saturation by agitation and aeration control.

After 64 hours growth, cells were treated in situ to kill them and to aid lysis. This was done by dropping the temperature to 15°C and adding sodium hydroxide to bring the pH to 10. After 50 minutes' treatment, the broth was readjusted to pH 7 with phosphoric acid and the cells harvested in a continuous centrifuge. The collected cells were distributed into different containers and stored at -20°C until required.

Frozen cells were thawed and resuspended at 25% w/v (based on wet cell weight) in 50 mM KH₂PO₄, pH 6.5. After stirring for 30 minutes, the cells were collected by centrifugation. Cells were then resuspended at 25% w/v (based upon wet cell weight) in lysis buffer, i.e. 0.3 M Na₂CO₃, pH 10.5. Lysis was conducted overnight at 4°C with stirring, after which the lysate was clarified by centrifugation to separate cell debris from the supernatant. The enzyme solution (i.e. lysate supernatant) was assayed for activity using standard biotransformation conditions, i.e. 1 ml enzyme solution suitably diluted in 0.1 M Na₂CO₃, pH 10.0, 40 µ 50% racemic ethyl ketoprofen, 0-5% Tween 80 stock solution, 2.5% w/v Triton x-100. Biotransformations were carried out in a sealed 20 ml volume glass vial with shaking for 1 hour at 25°C. Activity is expressed as U/ml activity where 1 unit (U) = 1 mg ketoprofen acid produced per hour at 25°C. Enzyme solutions were considered suitably diluted if the assayed activity was in the range of 1 to 5 U/ml.

A 50% racemic ethyl ketoprofen stock solution was prepared as follows: 25 g racemic ethyl ketoprofen and 0.25 g Tween 80 were added to 10 ml distilled water and this mixture sonicated for 5 mins (15 amplitude µm, 10 seconds on/10 seconds off cycle). The volume was then made up to 50 ml with distilled water, and the stock solution autoclaved to sterilise and allow for longer term storage.

A typical lysate activity was 1.5 U/ml.

### Example 5 Isolation and purification of hydrolytic activity

Clarified lysate (Example 4) was diafiltered against distilled water (Amicon DC2 ultra-filtration unit using 30,000 molecular weight cut off hollow fibre cartridges) until the conductivity of the lysate reached that of 20 mM Na₂CO₃, pH 9.2 buffer (Buffer A). The solution was then concentrated 4-5 fold and typically retained >90% initial activity. Enzyme activity was loaded batchwise onto pre-equilibrated QA52 anion exchange resin (Pharmacia) for 1 hour at ambient temperature, at a loading of 7-10 U of activity per ml of wet QA52 gel. The loaded gel was packed into a column at a linear flow rate of 200 mm/h. Chromatography was subsequently conducted at 4°C.

The column was washed to baseline with Buffer A. Elution was performed using a 10 column volume gradient of 0-05 M NaCl in Buffer A and fractions corresponding to 1/35 gradient volume were collected. The elution profile typically obtained is shown in Table 3.

**Table 3**

| ELUTION PROFILE FOR 1ST PASS QA52 CHROMATOGRAPHY | | |
|---|---|---|
| FRACTION | TOTAL ACTIVITY (U.) | RECOVERY (%) |
| LOAD | 99 | - |
| FLOW THROUGH | 0 | 0 |
| WASH | 0 | 0 |
| 1 | 0 | 0 |
| 5 | 0.3 | 0.3 |
| 6 | 2.0 | 2.0 |
| 7 | 6.6 | 6.7 |
| 8 | 19.5 | 19.7 |
| 9 | 22.2 | 22.4 |
| 10 | 10.0 | 10.1 |
| 11 | 2.5 | 2.5 |
| 13 | 1.0 | 1.0 |
| 17 | 0.3 | 0.3 |

Activity was eluted in the NaCl concentration range of 0.12-0.18 M. Fractions 7-10 were pooled inclusively to give:
pooled fraction activity recovery = 63.5 %
pooled fraction protein recovery = 15.8 %

The pooled activity was concentrated using a YM 30 membrane in a stirred cell ultrafiltration unit (AMICON) with no loss of activity, and dialysed against Buffer A until a conductivity of 3.2-3.6 mS was attained. This was then re-chromatographed on QA52 resin with the results shown in Table 4.

**Table 4**

| ELUTION PROFILE FOR 2ND PASS QA52 CHROMATOGRAPHY | | |
|---|---|---|
| FRACTION | TOTAL ACTIVITY (U) | RECOVERY (%) |
| LOAD | 3111 | - |
| FLOW THROUGH | 0 | 0 |
| WASH | 0 | 0 |
| 13 | 60 | 1.9 |
| 14 | 193 | 6.2 |
| 15 | 407 | 13.1 |
| 16 | 828 | 26.6 |
| 17 | 890 | 28.6 |
| 18 | 445 | 14.3 |
| 19 | 152 | 4.9 |
| 20 | 81 | 2.6 |

In this second pass, activity was eluted in the NaCl concentration range of 0.2-0.25 M NaCl. Fractions 15-17 were pooled inclusively. The pooled sample was assayed for activity and protein content.
Activity =27.8 U.ml⁻¹
Total activity = 2599 U
Total activity recovery = 83.5%

Protein (biuret method) = 1.34 mg.ml⁻¹
Total protein = 125 mg
Total protein recovery = 5.5 %

The pooled fractions were further purified using a Pharmacia MONO P HR 5/5 pre-packed anion exchange FPLC. The chromatographic conditions were as follows:
Low salt buffer = 20 mM Ethanolamine-HCl pH 9.0
High salt buffer = 20 mM Ethanolamine-HCL + 0.3M NaCl pH 9.0
Flow rate = 1 ml/min .

| Gradient profile | Time | NaCl concentration |
|---|---|---|
| | 0-5 mins | 0 M |
| | 5-7 mins | 0-0.1 M |
| | 7-20 mins | 0.1-0.3 M |
| | 20-22 mins | 0.3 M |
| | 22-23 mins | 0.3-0 M |
| | 23-25 mins | 0 M |

Sample volume = 1 ml
Fraction volume = 1 ml

The pooled fractions from the 2nd pass QA52 chromatography were concentrated 10 fold through a 30,000 molecular weight cut off ultrafiltration membrane and the resulting concentrate desalted into low salt buffer for HPLC, using a Pharmacia G-25 PD10 gel filtration column. Approximately 5 mg total protein were loaded onto the HPLC column per run. The following elution profile was obtained (Table 5):

**Table 5**

| ELUTION PROFILE FROM MONO P ANION EXCHANGE HPLC | | |
|---|---|---|
| FRACTION (mins) | ACTIVITY (U.ml⁻¹) | RECOVERY (%) |
| LOAD | 104.0 | - |
| 8 | 5.7 | 5.5 |
| 9 | 63.8 | 61.3 |
| 10 | 28.3 | 27.2 |
| 11 | 7.5 | 7.2 |
| 12 | 1.9 | 1.8 |
| 13 | 0.0 | 0.0 |

Fractions 9 and 10 were pooled to give a total activity recovery of 88.5%. This pooled activity was then further purified by size exclusion HPLC.

An Analgel-TSK G3000 SWXL 30 cm x 7.8 mm size exclusion HPLC column was used for the following separation. The following chromatographic conditions were employed:
Buffer - 0.1 M K₂HPO₄ + 20 mM Na₂EDTA pH 7.0
Flow rate = 0.4 ml.min⁻¹
Sample volume = 200 µl
Fraction volume = 400 µl

The pooled fractions from the MONO P ion exchange HPLC were concentrated 1.5 fold through a 10,000 molecular weight cut off ultrafiltration membrane . The concentrated sample was desalted into size exclusion HPLC buffer using a Pharmacia G25 pd10 column. The following elution profile was obtained (Table 6).

**Table 6**

| ELUTION PROFILE FROM SIZE EXCLUSION HPLC | | |
|---|---|---|
| FRACTION (mins) | ACTIVITY (U.ml⁻¹) | RECOVERY (%) |
| LOAD | 131.0 | - |
| 17.5 | 0.0 | 0 |
| 18.5 | 0.0 | 0 |
| 19.5 | 1.5 | 2.3 |
| 20.5 | 13.3 | 20.3 |
| 21.5 | 7.5 | 11.5 |
| 22.5 | 2.6 | 4.0 |
| 23.5 | 0.8 | 1.2 |

An estimation of the molecular weight of our AJ3 racemic ethyl ketoprofen esterase was made by SDS-PAGE (sodium dodecyl sulphate Polyacrylamide Gel Electrophoresis). A pre-cast 12% homogeneous SDS-PAGE mini-gel (BDH) was run using prestained molecular weight protein standard markers (BISC) for molecular weight comparison. The running Buffer was Electrograd Buffer TTS (BDH).

Electrophoresis was carried out at a constant voltage (200 V) and limiting current (40 mA per gel) until the blue dye front had passed completely through the gel. Protein was visualised by Coomassie Blue staining.

The band of protein corresponding to the esterase was identified by comparison of protein banding in active and non-active samples. Comparison of the relative migration distances of the active protein and the protein standards indicated the novel purified denatured protein had a molecular weight slightly greater than the 32,500 Da marker.

### Example 6 Effect of pH on the hydrolytic activity

The pH profile of the biotransformation was tested using the pooled material described above, following 4 months storage at -20°C. An aliquot was thawed at ambient temperature, and diluted 10-fold with distilled water (diluted enzyme solution). 1.1 M buffer stock solutions were prepared at the following pH, using the salts indicated (pH was adjusted as necessary using sodium hydroxyde or hydrochloric acid):

| | |
|---|---|
| pH 6.0 | NaH₂PO₄ |
| pH 7.0 | NaH₂PO₄ |
| pH 8.0 | NaH₂PO₄ |
| pH 9.0 | Glycine-HCl |
| pH 10.0 | Glycine-HCl |
| pH 11.0 | Na₂HPO₄ |

The biotransformation reactions were set up in sealed glass vials as follows:
1 ml diluted enzyme solution
100 µl relevant 1.1 M pH buffer stock solution
40 µl 50% racemic ethyl stock solution
2.5% w/v Triton X-100

For a biotransformation at pH 12.0, 1M NaOH solution was added directly to a pH 11.0 reaction mixture until the correct pH was reached. All biotransformations were set up in duplicate and reactions were carried out for 1 hour with shaking at 25°C. The main results of the reactions are shown in Table 7. Maximum activity was found at pH 10.

**TABLE 7**

| AJ3 BIOTRANSFORMATION pH PROFILE | | |
|---|---|---|
| BIOTRANSFORMATION pH | ACTIVITY (U.ml⁻¹) | NORMALISED RECOVERY (%) |
| 6 | 1.32 | 27.6 |
| 7 | 1.97 | 41.2 |
| 8 | 2.47 | 51.7 |
| 9 | 2.95 | 61.7 |
| 10 | 4.78 | 100.0 |
| 11 | 2.35 | 49.2 |
| 12 | 0.36 | 7.5 |

## Claims

1. A process for preparing predominantly (S)-2-(3-benzoylphenyl)propionic acid from a mixture of enantiomers of an alkyl ester of 2-(3-benzoylphenyl)propionic acid, which comprises using as a biocatalyst a microorganism of the genus Ophiostoma or Ceratocystis, or a material having enzymatic activiy derived therefrom.

2. A process according to claim 1, which gives (S)-2-(3-benzoylphenyl)propionic acid in at least 93% enantiomeric excess at 15-25% conversion.

3. A process according to claim 1 or claim 2, wherein the biocatalyst is the microorganism Ophiostoma novo-ulmi, IMI 356050.

4. A process according to claim 3, wherein the alkyl ester has 1 to 3 carbon atoms.

5. A process according to claim 4, wherein the alkyl ester is an ethyl ester.

6. A process according to any preceding claim, wherein the reaction is conducted at pH 8 to 11.

7. A process according to any preceding claim, wherein the reaction is conducted at pH 9.5 to 10.5.

## Patentansprüche

1. Verfahren zur überwiegenden Herstellung von (S)-2-(3-Benzoylphenyl)propionsäure aus einem Gemisch der Enantiomeren der Alkylester von 2-(3-Benzoylphenyl)propionsäure durch Verwendung eines Mikroorganismus des Genus Ophiostoma oder Ceratocystis oder eines Materials, welches sich davon ableitet und enzymatische Aktivität besitzt, als Biokatalysator.

2. Verfahren nach Anspruch 1, wobei die (S)-2-(3-Benzoylphenyl)propionsäure in mindestens 93%igem enantiomerem Überschuß bei einer 15 bis 25%igen Konversion erhalten wird.

3. Verfahren nach Anspruch 1 oder Anspruch 2, worin der Biokatalysator der Mikroorganismus Ophiostoma novo-ulmi, IMI 356050 ist.

4. Verfahren nach Anspruch 3, worin der Alkylester 1 bis 3 Kohlenstoffatome enthält.

5. Verfahren nach Anspruch 4, worin der Alkylester ein Ethylester ist.

6. Verfahren nach einem der vorhergehenden Ansprüche, worin die Reaktion bei einem pH von 8 bis 11 durchgeführt wird.

7. Verfahren nach einem der vorhergehenden Ansprüche, worin die Reaktion bei einem pH von 9.5 bis 10.5 durchgeführt wird.

## Revendications

1. Procédé pour préparer de façon prédominante de l'acide (S)-2-(3-benzoylphényl)-propionique à partir d'un mélange d'énantiomères d'un ester alkylique d'acide 2-(3-benzoylphényl)-propionique, qui comprend l'utilisation d'un microorganisme du genre *Ophiostoma* ou *Ceratocystis, ou* d'une matière ayant une activité enzymatique dérivée de celui-ci, en tant que biocatalyseur.

2. Procédé selon la revendication 1, qui donne l'acide (S)-2-(3-benzoylphényl)-propionique en un excès énantiomère d'au moins 93%, à une conversion de 15 à 25%.

3. Procédé selon les revendications 1 ou 2, dans lequel le biocatalyseur est le microorganisme *Ophiostoma novo-ulmi,* IMI 356050.

4. Procédé selon la revendication 3, dans lequel l'ester alkylique comprend 1 à 3 atomes de carbone.

5. Procédé selon la revendication 4, dans lequel l'ester alkylique est l'ester éthylique.

6. Procédé selon l'une quelconque des revendications précédentes, dans lequel la réaction est conduite à un pH de 8 à 11.

7. Procédé selon l'une quelconque des revendications précédentes, dans lequel la réaction est conduite à un pH de 9,5 à 10,5.
